# EUROPEAN PATENT APPLICATION

(11) **EP 2 671 588 A1**
(43) Date of publication of application: **11.12.2013**
(21) Application number: 12742258.2
(22) Date of filing: 01.02.2012
(51) Int. Cl.: A61K 31/496, A61K 9/70, A61K 47/06, A61K 47/10, A61K 47/12, A61K 47/14, A61K 47/22, A61K 47/24, A61K 47/32, A61K 47/34, A61K 47/44, A61P 25/18, A61P 43/00

(54) **TRANSDERMAL PATCH**

(30) Priority: 02.02.2011 JP 2011021197
(71) Applicant: Dainippon Sumitomo Pharma Co., Ltd., Osaka 541-8524 (JP)
(72) Inventor: MAEDA, Hiroo, Ibaraki-shi Osaka 567-0878 (JP); YAMAMOTO, Kazumitsu, Ibaraki-shi Osaka 567-0878 (JP); TANAKA, Masayasu, Ibaraki-shi Osaka 567-0878 (JP)
(74) Representative: Webster, Jeremy Mark
(86) International application number: PCT/JP2012/052312
(87) International publication number: WO 2012/105624

(57) **Abstract**

The present invention relates to an external preparation for transdermal administration, which remarkably enhances the skin permeability of 2-(4-ethyl-1-piperazinyl)-4-(4-fluorophenyl)-5,6,7,8,9,10-hexahydrocycloocta[b]pyridine (compound A). The adhesive preparation of the present invention has an adhesive layer formed on one surface of a support, and the adhesive layer contains (i) compound A or a physiologically acceptable acid addition salt thereof, (ii) an adhesive, (iii) lactic acid, and (iv) an additive containing a particular permeation enhancer, whereby remarkably superior skin permeability is provided.

## Description

### Technical Field

The present invention relates to an adhesive preparation for transdermal absorption. Specifically, the present invention relates to an adhesive preparation comprising 2-(4-ethyl-1-piperazinyl)-4-(4-fluorophenyl)-5,6,7,8,9,10-hexahydrocycloocta[b]pyridine (hereinafter sometimes to be referred to as "blonanserin" or compound A"), or a physiologically acceptable acid addition salt thereof as an active ingredient, which shows enhanced skin permeability of the compound when adhered to a skin surface, and can achieve a high blood drug concentration.

### Background Art

Blonanserin is a serotonin-dopamine antagonist (SDA) and is disclosed in patent document 1. Blonanserin shows high affinity for dopamine D2 and serotonin 5-HT2 receptor, as compared to haloperidol, and is useful as an antipsychotic agent.

Formulation of a drug such as blonanserin as an adhesive preparation for transdermal absorption is useful, since it can maintain blood drug concentration in a more sustained manner as compared to oral administration, and can lower liver metabolism and drug interaction by avoiding first pass effect. In addition, administration by way of a transdermal absorption preparation has many advantages such as absence of influence of diet, possible administration to patients having difficulty in swallowing, easy confirmation and discontinuation of medication and the like.

Under the circumstances, a preparation for transdermal administration of blonanserin, which suppresses production of metabolites and can maintain blood drug concentration in a sustained manner, is described in patent document 2. Specifically, it shows enhanced skin permeability of blonanserin, which is achieved by a particular permeation enhancer contained therein.

In terms of actual medical treatments, that is, for the significance of improving the quality-of-life (QOL) of patients, however, reduction of foreign-body feeling and uncomfortable feeling caused by the adhesion of a transdermal absorption adhesive preparation to a skin surface is very important. Accordingly, the area of adhesion, that is, the size of the adhesive preparation for transdermal absorption, is desired to be as small as possible. For this end, it is also important to further improve the skin permeability.

### [Document List]

### [patent documents]

patent document 1: JP-B-H7-47574
patent document 2: WO2007/142295

### SUMMARY OF THE INVENTION

### Problems to be Solved by the Invention

The present invention aims to provide an adhesive preparation for transdermal administration, which can remarkably enhance the skin permeability of blonanserin. Means of Solving the Problems

The present inventors have intensively studied a composition that enhances skin permeation of blonanserin and found that, when lactic acid and a particular additive having such effect but showing a lower blonanserin permeability-enhancing effect as compared to lactic acid are combined, the amount of skin permeation of blonanserin becomes remarkably high as compared to the total permeation amount of respective preparations containing each of them singly as a permeation enhancer (preparation containing lactic acid alone and preparation containing the above-mentioned particular additive alone), that is, a synergistic effect on the enhancement of permeation can be afforded by a combination of lactic acid and the particular additive, which resulted in the completion of the present invention.
Accordingly, the present invention provides the following.

[1] An adhesive preparation comprising a support and an adhesive layer formed on one surface of the support, wherein the adhesive layer comprises (i) 2-(4-ethyl-1-piperazinyl)-4-(4-fluorophenyl)-5,6,7,8,9,10-hexahydrocycloocta[b]pyridine or a physiologically acceptable acid addition salt thereof, (ii) an adhesive, (iii) lactic acid, and (iv) at least one kind of additive selected from the group consisting of polyethylene glycol mono-p-isooctylphenylether, diisopropyl adipate, α-monoisostearyl glyceryl ether, oleic acid, acetic acid, propylene glycol, sesame oil, lauromacrogol, crotamiton, sorbitan monostearate, cetyl lactate, lauryl alcohol, cetyl alcohol, diethyl sebacate, sorbitan sesquioleate, egg-yolk lecithin, propylene carbonate, liquid paraffin, polyoxyethylene hydrogenated castor oil, polyethylene glycol monostearate, octyl alcohol and benzyl alcohol.
[2] The adhesive preparation of the above-mentioned [1], wherein the additive is at least one kind selected from the group consisting of polyethylene glycol mono-p-isooctylphenylether, diisopropyl adipate, α-monoisostearyl glyceryl ether, oleic acid, acetic acid, propylene glycol, sesame oil, and lauromacrogol.
[3] The adhesive preparation of the above-mentioned [1] or [2], wherein the additive is at least one kind selected from the group consisting of diisopropyl adipate, α-monoisostearyl glyceryl ether, oleic acid, propylene glycol, sesame oil, and lauromacrogol.
[4] The adhesive preparation of any of the above-mentioned [1] - [3], wherein the additive comprises α-monoisostearyl glyceryl ether.
[5] The adhesive preparation of any of the above-mentioned [1] - [4], wherein the additive comprises sesame oil.
[6] The adhesive preparation of any of the above-mentioned [1] - [5], wherein the additive comprises lauromacrogol.
[7] The adhesive preparation of any of the above-mentioned [1] - [6], wherein the additive comprises diisopropyl adipate.
[8] The adhesive preparation of any of the above-mentioned [1] - [7], wherein the additive comprises propylene glycol.
[9] The adhesive preparation of any of the above-mentioned [1] - [8], wherein the additive comprises oleic acid.
[10] The adhesive preparation of the above-mentioned [1] or [2], wherein the additive comprises polyethylene glycol mono-p-isooctylphenylether.
[11] The adhesive preparation of any of the above-mentioned [1] - [10], wherein the content of lactic acid in the adhesive layer is 0.01 - 20 wt%.
[12] The adhesive preparation of any of the above-mentioned [1] - [11], wherein the additive is contained at a ratio of 0.5 - 20 parts by weight per 1 part by weight of the lactic acid.
[13] The adhesive preparation of the above-mentioned [12], wherein the additive is contained at a ratio of 0.8 - 12 parts by weight per 1 part by weight of the lactic acid.
[14] The adhesive preparation of any of the above-mentioned [1] - [13], wherein the total content of lactic acid and the additive in the adhesive layer is 0.1 - 60 wt%.
[15] The adhesive preparation of any of the above-mentioned [1] - [14], wherein the adhesive is at least one kind selected from an acrylic adhesive, a rubber adhesive and a silicone adhesive.
[16] The adhesive preparation of the above-mentioned [15], wherein the adhesive comprises an acrylic adhesive.
[17] The adhesive preparation of the above-mentioned [15] or [16], wherein the acrylic adhesive is at least one kind selected from the group consisting of a (co)polymer mainly comprising (meth)acrylic acid alkyl ester, and a copolymer of (meth)acrylic acid alkyl ester and a functional monomer.
[18] The adhesive preparation of the above-mentioned [15] or [16], wherein the adhesive comprises a rubber adhesive.
[19] The adhesive preparation of the above-mentioned [15] or [18], wherein the rubber adhesive is at least one kind selected from the group consisting of a styrene-isoprene-styrene block copolymer and polyisobutylene.
[20] The adhesive preparation of any of the above-mentioned [1] - [19], wherein the adhesive layer comprises component (i) at a concentration of 0.5 - 40 wt% in terms of compound A.
[21] The adhesive preparation of any of the above-mentioned [1] - [20] whose target disease is schizophrenia.
[22] A method for transdermally administering 2-(4-ethyl-1-piperazinyl)-4-(4-fluorophenyl)-5,6,7,8,9,10-hexahydrocycloocta[b]pyridine, comprising applying the adhesive preparation of any one of the above-mentioned [1] to [20] to a skin of a patient.
[23] A method for treating schizophrenia, comprising applying the adhesive preparation of any one of the above-mentioned [1] to [20] to a skin of a patient with schizophrenia.

### Effect of the Invention

The adhesive preparation for transdermal absorption of the present invention shows high permeability, and therefore, it can provide a small-sized preparation when providing an adhesive preparation required in the medical practice for mental diseases such as schizophrenia and the like, and can further reduce the content of blonanserin in the preparation. Therefore, it can contribute to the improvement of usability in medical care and economic efficiency.

### Description of Embodiments

Preferable embodiments of the present invention are explained in detail in the following.

In the present invention, an adhesive preparation means a preparation in general, which can be adhered to the skin, including, for example, tape preparations, patch preparations, cataplasm preparations, plaster preparation and the like.

In the present claims and the present specification, the adhesive layer is a layer formed on a support, which comprises a drug. It contains at least (i) compound A or a physiologically acceptable acid addition salt thereof, (ii) an adhesive, (iii) lactic acid, and (iv) an additive, and may further contain other component for formulation.

In the present claims and the present specification, those simply shown by "wt%" mean wt% when the total weight of the adhesive layer substantially free of solvent and the like by drying and the like is 100 wt%.

### (i) Compound A or a physiologically acceptable acid addition salt thereof

Compound A in the present invention, i.e., 2-(4-ethyl-1-piperazinyl)-4-(4-fluorophenyl)-5,6,7,8,9,10-hexahydrocycloocta[b]pyridine (generic name "blonanserin") is a compound represented by the following formula:

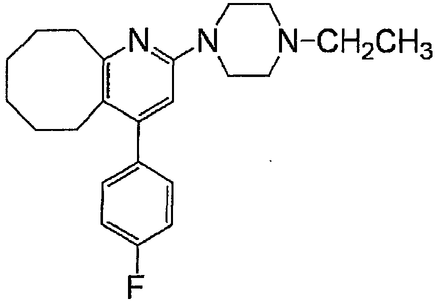

, which is a serotonin-dopamine antagonist (or dopamine-serotonin antagonist) as mentioned above, and is commercially available as an antipsychotic agent.

Compound A may be a free base or a physiologically acceptable acid addition salt thereof. Examples of an addition salt of organic acid include, but are not limited to, formate, acetate, lactate, adipate, citrate, tartrate, methanesulfonate, fumarate, maleate and the like, and examples of an addition salt of inorganic acid include, but are not limited to, hydrochloride, sulfate, nitrate, phosphate and the like. Furthermore, compound A or a physiologically acceptable acid addition salt thereof may also be a solvate, or a hydrate or non-hydrate.

The above-mentioned compound A or a physiologically acceptable acid addition salt thereof can be produced according to, for example, the method described in JP-B-H7-47574 (patent document 1) or a method analogous thereto. The produced compound A or a physiologically acceptable acid addition salt thereof may be pulverized as appropriate according to a conventional method.

The amount of "compound A or a physiologically acceptable acid addition salt thereof" contained in the adhesive preparation of the present invention is generally not less than about 0.5 wt%, preferably, not less than 1 wt%, not more than 40 wt%, preferably not more than 30 wt%, more preferably not more than 20 wt%, generally about 0.5 - about 40 wt%, in terms of compound A, of an adhesive layer as 100 wt%, and it is preferably about 0.5 - about 30 wt%, more preferably about 0.5 - about 20 wt%, and preferably about 1 - about 40 wt%, more preferably about 1 - about 30 wt%, particularly preferably about 1 - about 20 wt%, in terms of compound A, of an adhesive layer as 100 wt%, though subject to change depending on the area of the adhesive preparation. Here, "in terms of compound A" means that, when compound A is in the form of a physiologically acceptable acid addition salt or compound A contains crystal water, the amount corresponding to the acid added to the acid addition salt or the crystal water is not included in the weight of compound A. In other words, it means that the amount of a physiologically acceptable acid addition salt of compound A or a hydrate thereof is calculated by converting the weight thereof to the weight of an equimolar amount of compound A (free base non-hydrate).

### (ii) Adhesive

Examples of the adhesive of the present invention include polymer adhesives such as silicone adhesives, rubber adhesives, acrylic adhesives and the like.

Examples of the silicone adhesive include those containing silicone rubber as a main component, such as polydimethyl siloxane, diphenylsiloxane and the like. Examples of the rubber adhesive include natural rubber, polyisopropylene rubber, polyisobutylene, styrene-butadiene copolymer, styrene-isopropylene copolymer, styrene-isoprene-styrene block copolymer and the like.

Examples of the acrylic adhesive include (co)polymers mainly comprising (meth)acrylic acid alkyl ester. Specific examples include polymers mainly comprising acrylic acid alkyl ester, polymers mainly comprising methacrylic acid alkyl ester, copolymers mainly comprising acrylic acid alkyl ester, copolymers mainly comprising methacrylic acid alkyl ester, and copolymers mainly comprising acrylic acid alkyl ester and methacrylic acid alkyl ester. The (co)polymer may be a copolymer of two or more kinds of (meth)acrylic acid alkyl ester as mentioned above, or may be a copolymer of (meth)acrylic acid alkyl ester and (a) functional monomer(s) capable of copolymerizing with (meth)acrylic acid alkyl ester.
Here, the "(meth)acrylic acid" means "acrylic acid or methacrylic acid", or "acrylic acid and/or methacrylic acid", and the "(co)polymer" means "polymer or copolymer", or "polymer and/or copolymer".

Examples of the (meth)acrylic acid alkyl ester include those obtained by esterification with straight chain or branched chain alkyl having a carbon number of 1 - 18, and specifically include (meth)acrylic acid methyl ester, (meth)acrylic acid butyl ester, (meth)acrylic acid hexyl ester, (meth)acrylic acid octyl ester, (meth)acrylic acid nonyl ester, (meth)acrylic acid decyl ester and the like. Examples of the functional monomer include a monomer having a hydroxyl group ((meth)acrylic acid hydroxyethyl ester etc.), a monomer having a carboxyl group (butyl maleate, crotonic acid etc.), a monomer having an amido group ((meth)acrylamide etc.), a monomer having an amino group (dimethylaminoacrylic acid ester etc.), a monomer having a pyrrolidone ring (N-vinyl-2-pyrrolidone etc.) and the like.

The acrylic adhesive in the present invention may be used singly or in a combination of two or more kinds thereof. In addition, it may be a mixture with other adhesive. Examples of the other adhesive include silicone adhesive, rubber adhesive and the like.

Specific preferable examples of the acrylic adhesive include, but are not limited to, acrylic acid-acrylic acid octyl ester copolymer, acrylic acid ester-vinyl acetate copolymer, 2-ethylhexyl acrylate-vinylpyrrolidone copolymer, 2-ethylhexyl acrylate-2-ethylhexyl methacrylate-dodecyl methacrylate copolymer, ethyl acrylate-methyl methacrylate copolymer, acrylic acid-silk fibroin copolymer, methyl acrylate-2-ethylhexyl acrylate copolymer and the like, and include commercially available products such as "POLYTHICK 410-SA" manufactured by Sanyo Chemical Industries, Ltd., "Oribain BPS-4849-40" manufactured by TOYO INK CO., LTD., "DURO-TAK 87-2194 (registered trade mark)" and "DURO-TAK387-2516 (registered trade mark)" manufactured by National Starch and Chemical Corp., "MAS811", "MAS683" and "MAS955" manufactured by CosMED Pharmaceutical Co. Ltd. and the like.

To afford appropriate adhesiveness to the skin, moreover, a curing agent may also be added where necessary. Examples of the curing agent include commercially available products such as "POLYTHICK SC-75" manufactured by Sanyo Chemical Industries, Ltd., "BHS8515" manufactured by TOYO INK CO., LTD. and the like. The amount thereof to be added can be appropriately selected according to the property of the adhesive, which is, for example, about 0.001 - 0.05 part by weight relative to 1 part by weight of the adhesive.

The amount of the adhesive to be added is a balance after removing (i) compound A or a physiologically acceptable acid addition salt thereof, (iii) lactic acid, (iv) the following particular additives, and various components mentioned below, which are added for formulation as necessary, from the adhesive layer, and is the amount necessary for completing the adhesive layer. Thus, for example, when the adhesive layer comprises about 10 wt% of compound A and about 20 wt% of lactic acid and the following particular additive in total, the amount of the adhesive is about 70 wt%.

The adhesiveness of the adhesive to be used here is of the level employed for medical adhesive preparations, and intended to mean adhesiveness of the level permitting easy adhesion to the skin and causing no particular problem in peeling off.

### (iii) Lactic acid

The lactic acid may be a DL-lactic acid (racemate), and may also be L-lactic acid or D-lactic acid (optically active forms). The lactic acid in the present specification may be either of them.

The amount of the lactic acid to be contained in the adhesive preparation of the present invention is not less than about 0.01 wt%, preferably not less than about 0.1 wt%, not more than about 20 wt%, preferably not more than about 15 wt%, more preferably not more than about 10 wt%, and generally about 0.01 - about 20 wt%, preferably about 0.1 - about 15 wt%, more preferably about 0.1 - about 10 wt%, of an adhesive layer as 100 wt%.

### (iv) Additive

In an adhesive preparation for transdermal absorption, which contains compound A, it has been found that, when lactic acid and a particular additive having a lower compound A permeability-enhancing effect as compared to lactic acid are contained in combination, the permeation amount of compound A becomes remarkably high as compared to the total permeation amount of a preparation containing lactic acid alone and a preparation containing the above-mentioned particular additive alone, that is, a synergistic effect on the enhancement of permeation can be afforded by a combination of lactic acid and the particular additive.

Examples of the preferable particular additive to be used in the present invention include, but are not limited to, polyethylene glycol mono-p-isooctylphenylether, diisopropyl adipate, α-monoisostearyl glyceryl ether, oleic acid, acetic acid, propylene glycol, sesame oil, lauromacrogol, crotamiton, sorbitan monostearate, cetyl lactate, lauryl alcohol, cetyl alcohol, diethyl sebacate, sorbitan sesquioleate, egg-yolk lecithin, propylene carbonate, liquid paraffin, polyoxyethylene hydrogenated castor oil, polyethylene glycol monostearate, octyl alcohol, benzyl alcohol and the like. Preferred are polyethylene glycol mono-p-isooctylphenylether, diisopropyl adipate, α-monoisostearyl glyceryl ether, oleic acid, acetic acid, propylene glycol, sesame oil, lauromacrogol, crotamiton, sorbitan monostearate, cetyl lactate, lauryl alcohol and cetyl alcohol, more preferred are polyethylene glycol mono-p-isooctylphenylether, diisopropyl adipate, α-monoisostearyl glyceryl ether, oleic acid, acetic acid, propylene glycol, sesame oil and lauromacrogol. Among these, diisopropyl adipate, α-monoisostearyl glyceryl ether, oleic acid, propylene glycol, sesame oil and lauromacrogol are still more preferable.
The sesame oil that may be used in the present invention is an oil obtained by squeezing sesame seeds, which may be an oil derived from raw sesame seeds, an oil derived from roasted sesame seeds or a mixture of the both. It is possible to use "sesame oil", which is a commercially available product for food, medicine and the like.

The above-mentioned particular additive may be used alone, or two or more kinds thereof may be used in combination. Particularly, for example, sesame oil alone, or a combination of sesame oil and α-monoisostearyl glyceryl ether is preferable.

The amount of the particular additive is generally 0.5 - 20 parts by weight, preferably 0.8 - 12 parts by weight, per 1 part by weight of the lactic acid. The amount of the particular additive to be added to the adhesive preparation of the present invention is generally about 0.1 - about 60 wt%, preferably about 0.1 - about 40 wt%, more preferably about 0.3 - about 40 wt%, as a total with the lactic acid, in 100 wt% of the adhesive layer.

### Components for formulation

The adhesive layer in the adhesive preparation of the present invention may comprise, as long as no particular problem is caused, a pharmaceutically acceptable component used for formulating an adhesive preparation. As such component, any component can be used as long as its addition does not cause any particular problem and the addition is necessary, and, for example, stabilizer, tackifier, plasticizer, flavor, filler, thickener, curing agent and the like can be mentioned.

Examples of the stabilizer include, but are not limited to, ascorbic acid, sodium alginate, propyleneglycol alginate, dibutylhydroxytoluene, butylhydroxyanisole, tocopherol acetate, tocopherol, propyl gallate, ethyl p-hydroxybenzoate, butyl p-hydroxybenzoate, propyl p-hydroxybenzoate, methyl p-hydroxybenzoate, 2-mercaptobenzimidazole and the like.

Examples of the tackifier include, but are not limited to, ester gum, glycerol, glycerol ester of hydrogenated rosin, petroleum resin, rosin, polybutene and the like. Examples of the plasticizer include, but are not limited to, polybutene, glycerol, glycerin fatty acid ester and the like. Examples of the flavor include, but are not limited to, dl-menthol, orange oil, peppermint oil, lemon oil, rose oil and the like. Examples of the filler include, but are not limited to, titanium oxide, zinc oxide, acrylic acid starch 100 and the like.

Examples of the thickener include, but are not limited to, carboxymethylcellulose, carrageenan, pectin, poly-N-vinylacetamide, copolymer of N-vinylacetamide and sodium acrylate and the like.

In addition, the pharmaceutically active ingredient in the adhesive layer may comprise other pharmaceutically active ingredients other than compound A. For example, other antipsychotic agents such as haloperidol, clozapine, risperidone, olanzapine, quetiapine, ziprasidone, aripiprazole, asenapine, amisulpride, iloperidone, cariprazine, sertindole, zotepine, paliperidone, bifeprunox and the like, and the like may be contained.

### Adhesive preparation of the present invention

The adhesive preparation of the present invention comprises a support, the aforementioned adhesive layer formed on one surface (one side) of the support, and a release liner as appropriate on the other surface free of contact with the support of the adhesive layer. When in use, the release liner is peeled off and the adhesive layer of the adhesive preparation is adhered to the skin, whereby transdermal administration is performed.

The adhesive preparation of the present invention is a systemic action type external preparation expected to provide efficacy by delivering the transdermally absorbed active ingredient to the systemic blood flow

The adhesive preparation of the present invention includes tape preparation, patch preparation, cataplasm preparation, plaster preparation and the like, as mentioned above, with preference given to tape preparation and patch preparation.

The support is not particularly limited as long as it is made of a material impermeable to drugs or hardly permeable to drugs, and uninfluential on drug release or hardly influential on drug release, and may be stretchable or nonstretchable. Examples thereof include, but are not limited to, resin film such as ethylcellulose, nylon, polyethylene terephthalate (PET), polyester, polypropylene and the like, and a combination thereof. In addition, non-woven fabric such as PET and the like may be formed on one side of a support on which an adhesive layer is not formed. Furthermore, it may be a support with a single layer structure or a structure of a laminate of plural materials. The support may be colorless and transparent, may be colored white or flesh color and the like. The support colored white or flesh color and the like may have a surface of the support coated with a dye or may have a support containing dye, pigment or the like uniformly kneaded therein.

The support surface on which an adhesive layer is formed is preferably subjected to, for example, a surface treatment such as corona discharge treatment, plasma treatment, oxidation treatment, hairline processing, sand mat processing and the like.

The adhesive preparation of the present invention can be produced by a conventional method. For example, the preparation can be produced according to the section relating to the production of a plaster preparation described in "Manual for the development of transdermal formulation" Edited by Mitsuo Matsumoto (1985) and the like. In addition, for example, the preparation can be produced by using the apparatus, method and the like described in "Development of Apparatus for Producing Adhesive Preparation for Transdermal Treatment System (MEMBRANE, 32(2), 116-119 (2007))".

To be specific, in the production of the adhesive preparation of the present invention, a general production method of adhesive tapes can be applied to form an adhesive layer. Representative example thereof is a solvent coating method. Besides this, a hot-melt coating method, an electron beam cured emulsion coating method and the like can be used.

When the adhesive layer is formed by a solvent coating method, for example, an adhesive layer mixture is prepared by mixing compound A or an acid addition salt thereof, a mixture comprising an adhesive, lactic acid and an additive and, where necessary, a formulation component such as a curing agent and the like, and an organic solvent, the mixture is applied onto one surface of a support or a release liner, the organic solvent is removed by drying, and a release liner or a support is adhered at a certain timing before or after drying. The thickness of the obtained adhesive layer is within the range of about 10 - about 400 µm, preferably about 20 - about 200 µm. However, the thickness of the adhesive layer is not limited to these ranges, and any thickness greater or smaller than these ranges is within the scope of the present invention.

The release liner to cover the surface of the adhesive layer is appropriately selected. Examples of the release liner include, but are not limited to, a release liner having a release layer having detachability on the surface thereof, such as a paper liner treated with a silicone resin and the like, a plastic film and the like.

The thus-obtained adhesive preparation of the present invention may be produced to have a suitable size in consideration of the factors such as dose and the like, or cut into such form. An adhesive preparation with such size may be a tape having a size larger than the size to be actually adhered, or conversely smaller, and may be cut as appropriate when in use or a suitable number thereof may be adhered in a row. While the part of the body to which the preparation is applied is not particularly limited, for example, arm, shoulder, cervical part, back, waist, abdomen, thorax, hip, leg and the like can be mentioned. The adhesive preparation of the present invention is packed with a written matter containing information relating to the adhesive preparation and distributed. The written matter may be placed on a package or contained in a package as an instruction sheet. Examples of the "information relating to the adhesive preparation" here include information stating that the preparation can or should be used for the treatment of schizophrenia.

### Examples

The present invention is explained in more detail in the following by referring to Examples, Reference Examples, Experimental Examples and the like, which are not to be construed as limitative. In the following Examples and the like, "%" means "wt%".

As a support, a 25 µm polyethylene terephthalate film manufactured by FUJIMORI KOGYO CO., LTD. was used. As a release liner, a Bynasheet 64S-018B manufactured by FUJIMORI KOGYO CO., LTD. was used. As lactic acid, DL-lactic acid was used.

### Example 1

An acrylic adhesive (POLYTHICK 410-SA, manufactured by Sanyo Chemical Industries, Ltd., solid content 38%) (4.25 g), a curing agent (POLYTHICK SC-75, manufactured by Sanyo Chemical Industries, Ltd., solid content 75 wt%) (9.0 mg), ethyl acetate (1.2 mL), and lactic acid and polyethylene glycol mono-p-isooctylphenylether were respectively added in such amounts that would achieve the content percentage thereof in the adhesive layer of 5%. To the mixture was added compound A such that its content percentage in the adhesive layer was 9%, and the mixture was sufficiently stirred. The obtained mixture was spread on the support such that the thickness of the adhesive layer after drying was about 60 µm, and the layer was dried at room temperature for one day. Then, a release liner was adhered to give a tape preparation.

### Examples 2 - 22

Tape preparations were produced using various additives shown in Table 1 instead of polyethylene glycol mono-p-isooctylphenylether in Example 1.

### Reference Example 1

An acrylic adhesive (POLYTHICK 410-SA, manufactured by Sanyo Chemical Industries, Ltd., solid content 38%) (4.25 g), a curing agent (POLYTHICK SC-75, manufactured by Sanyo Chemical Industries, Ltd., solid content 75 wt%) (9.0 mg), ethyl acetate (1.2 mL), and polyethylene glycol mono-p-isooctylphenylether was added such that the content percentage thereof in the adhesive layer was 10%. To the mixture was added compound A such that its content percentage in the adhesive layer was 9%, and the mixture was sufficiently stirred. The obtained mixture was spread on the support such that the thickness of the adhesive layer after drying was about 60 µm, and the layer was dried at room temperature for one day. Then, a release liner was adhered to give a tape preparation.

### Reference Examples 2 - 25

Tape preparations were produced in the same manner by using various additives shown in Table 1 instead of polyethylene glycol mono-p-isooctylphenylether in Reference Example 1.

### Comparative Examples 1 and 2

Tape preparations were produced using glycerol or cetanol - polyethylene glycol monostearate mixed wax instead of polyethylene glycol mono-p-isooctylphenylether in Example 1 (Comparative Example 1, Comparative Example 2).

### Experimental Example 1

### Hairless rat skin permeation experiment

Using an in vitro diffusion cell, the skin permeability of compound A in the tape preparations obtained in Examples 1 - 22, Reference Examples 1 - 25, and Comparative Examples 1, 2 through the abdominal skin of 5- to 6-week-old hairless rats was measured. That is, the skin of hairless rat was set on an in vitro diffusion cell with permeation area 1.13 cm² and using, as a receiver fluid, 0.75 mL of a 2:1 mixture of polyethylene glycol 200 (macrogol 200) and phosphate buffer, each preparation was adhered to the skin on the donor side (n=4). The receiver fluid was stirred for 24 hr while keeping at 37°C, the concentration of compound A in the receiver fluid was measured by high performance liquid chromatography (column: YMC AM312 ODS 5 µm (6 mmϕ×150 mm; YMC), mobile phase: 0.01 mol/l dodecylsulfuric acid Na-containing aqueous solution (adjusted to pH 2.4 with phosphoric acid):acetonitrile:methanol (2:5:3), column temperature: 40°C, flow rate: 1.0 mL/min), and the permeation amount of compound A in each preparation was determined. The results are shown in Table 1.

**Table 1**

| additive | permeation amount of compound A in hairless rat skin permeation test (µg/cm²/24 hr) | | synergistic effect on permeability by combination of lactic acid and additive (note) |
|---|---|---|---|
| | Ref. Ex. (additive 10% alone (b)) | Ex. (lactic acid 5% and additive 5% (c)) | |
| polyethylene glycol mono-p-isooctylphenylether | 0.37 [Ref. Ex. 1] | 13.7 [Ex. 1] | 3.9 |
| diisopropyl adipate | 0.25 [Ref. Ex. 2] | 13.3 [Ex. 2] | 3.8 |
| α-monoisostearyl glyceryl ether | 0.32 [Ref. Ex. 3] | 12.6 [Ex. 3] | 3.6 |
| oleic acid | 0.21 [Ref. Ex. 4] | 12.5 [Ex. 4] | 3.6 |
| acetic acid | 0.25 [Ref. Ex. 5] | 10.6 [Ex. 5] | 3.1 |
| propylene glycol | 0.33 [Ref. Ex. 6] | 10.2 [Ex. 6] | 2.9 |
| sesame oil | 1.05 [Ref. Ex. 7] | 10.2 [Ex. 7] | 2.6 |
| lauromacrogol | 0.53 [Ref. Ex. 8] | 10.0 [Ex. 8] | 2.8 |
| crotamiton | 0.28 [Ref. Ex. 9] | 9.3 [Ex. 9] | 2.7 |
| sorbitan monostearate | 0.16 [Ref. Ex. 10] | 9.3 [Ex. 10] | 2.7 |
| cetyl lactate | 0.34 [Ref. Ex. 11] | 9.0 [Ex. 11] | 2.6 |
| lauryl alcohol | 0.59 [Ref. Ex. 12] | 8.8 [Ex. 12] | 2.4 |
| cetyl alcohol | 0.17 [Ref. Ex. 13] | 8.7 [Ex. 13] | 2.5 |
| diethyl sebacate | 0.33 [Ref. Ex. 14] | 8.2 [Ex. 14] | 2.3 |
| sorbitan sesquioleate | 0.26 [Ref. Ex. 15] | 8.1 [Ex. 15] | 2.3 |
| egg-yolk lecithin | 0.14 [Ref. Ex. 16] | 7.9 [Ex. 16] | 2.3 |
| propylene carbonate | 0.17 [Ref. Ex. 17] | 7.9 [Ex. 17] | 2.3 |
| liquid paraffin | 0.17 [Ref. Ex. 18] | 7.5 [Ex. 18] | 2.2 |
| polyoxyethylene hydrogenated castor oil | 0.13 [Ref. Ex. 19] | 7.4 [Ex. 19] | 2.2 |
| polyethylene glycol monostearate | 0.05 [Ref. Ex. 20] | 7.2 [Ex. 20] | 2.1 |
| octyl alcohol | 0.18 [Ref. Ex. 21] | 7.2 [Ex. 21] | 2.1 |
| benzyl alcohol | 0.16 [Ref. Ex. 22] | 6.8 [Ex. 22] | 2.0 |
| glycerol | 0.56 [Ref. Ex. 23] | 3.7 [Comp. Ex. 1] | 1.02 |
| cetanol - polyethylene glycol monostearate mixed wax | 0.39 [Ref. Ex. 24] | 1.3 [Comp. Ex. 2] | 0.37 |
| lactic acid | 6.70(a) [Ref. Ex. 25] | | |

| | | | |
|---|---|---|---|
| (note) The "synergistic effect on permeability by a combination of lactic acid and an additive" is a value obtained by dividing "permeation amount (c) of compound A in a preparation added with lactic acid 5% and additive 5% in combination" by the average of "permeation amount (a) of compound A in a preparation added with lactic acid 10% alone" and "permeation amount (b) of compound A in a preparation added with additive 10% alone", i.e., (c) ÷ Ave[(a),(b)]. | | | |

As shown in Table 1, the obtained permeability data reveals that the value of "a synergistic effect of a combination of lactic acid and an additive on the permeability", namely, (c) ÷ Ave[(a),(b)], is clearly larger than 1. This exhibits the superior feature of the present invention that a combination of lactic acid and the additive synergistically enhances the skin permeability of compound A. Therefrom it has been clarified that the skin permeability of compound A in the adhesive preparation is particularly remarkably improved when the additive used is one used in Examples 1 - 22 and the like, which is a combination of lactic acid and a particular additive.

### Examples 23 - 29

In the same manner as in Example 1, tape preparations were produced using respective starting materials described in Table 2.
For example, in Example 23 of Table 2, an acrylic adhesive (POLYTHICK 410-SA, manufactured by Sanyo Chemical Industries, Ltd., solid content 38%) (3.72 g), a curing agent (POLYTHICK SC-75, manufactured by Sanyo Chemical Industries, Ltd., solid content 75 wt%) (7.0 mg), ethyl acetate (1.2 mL), and lactic acid and sesame oil were added in such amounts that would achieve the content percentage thereof in the adhesive layer of 4% and 16%, respectively. To the mixture was added compound A such that its content percentage in the adhesive layer was 9%, and the mixture was sufficiently stirred. The obtained mixture was spread on the support such that the thickness of the adhesive layer after drying was about 60 µm, and the layer was dried at room temperature for one day. Then, a release liner was adhered to give a tape preparation.

### Reference Examples 26 - 33

In the same manner as in Example 1, tape preparations were produced using respective starting materials described in Table 2.
For example, in Reference Example 27 of Table 2, an acrylic adhesive (POLYTHICK 410-SA, manufactured by Sanyo Chemical Industries, Ltd., solid content 38 wt%) (3.93 g), a curing agent (POLYTHICK SC-75, manufactured by Sanyo Chemical Industries, Ltd., solid content 75 wt%) (8.0 mg), ethyl acetate (1.2 mL), and sesame oil was added such that would achieve the content percentage thereof in the adhesive layer of 16%. To the mixture was added compound A such that its content percentage in the adhesive layer was 9%, and the mixture was sufficiently stirred. The obtained mixture was spread on the support such that the thickness of the adhesive layer after drying was about 60 µm, and the layer was dried at room temperature for one day. Then, a release liner was adhered to give a tape preparation.

**Table 2**

| | acrylic adhesive (g) | curing agent (mg) | ethyl acetate (mL) | content percentage (%) of lactic acid in adhesive layer | content percentage (%) of sesame oil in adhesive layer | content percentage (%) of compound A in adhesive layer |
|---|---|---|---|---|---|---|
| Ref. Ex. 26 | 4.56 | 9 | 1.2 | 4 | - | 9 |
| Ex. 23 | 3.72 | 7 | 1.2 | 4 | 16 | 9 |
| Ref. Ex. 27 | 3.93 | 8 | 1.2 | - | 16 | 9 |
| Ex. 24 | 3.30 | 7 | 1.2 | 4 | 24 | 9 |
| Ref. Ex. 28 | 3.51 | 7 | 1.2 | - | 24 | 9 |
| Ex. 25 | 2.88 | 6 | 1.2 | 4 | 32 | 9 |
| Ref. Ex. 29 | 3.09 | 6 | 1.2 | - | 32 | 9 |
| Ex. 26 | 2.67 | 5 | 1.2 | 4 | 36 | 9 |
| Ref. Ex. 30 | 2.88 | 6 | 1.2 | - | 36 | 9 |
| Ex. 27 | 2.46 | 5 | 1.2 | 4 | 40 | 9 |
| Ref. Ex. 31 | 2.67 | 5 | 1.2 | - | 40 | 9 |
| Ex. 28 | 2.25 | 5 | 1.2 | 4 | 44 | 9 |
| Ref. Ex. 32 | 2.46 | 5 | 1.2 | - | 44 | 9 |
| Ex. 29 | 2.05 | 4 | 1.2 | 4 | 48 | 9 |
| Ref. Ex. 33 | 2.25 | 5 | 1.2 | - | 48 | 9 |

### Experimental Example 2

In the same manner as in Experimental Example 1 and using an in vitro diffusion cell, the skin permeability of compound A in the tape preparations obtained in Examples 23 - 29, Reference Example 26 - 33 through the abdominal skin of hairless rats was measured. The results are shown in Table 3.

**Table 3**

| | composition in adhesive layer | | | permeation amount of compound A in hairless rat skin permeation test (µg/cm²/24 hr) | |
|---|---|---|---|---|---|
| | lactic acid content percentage (%) | sesame oil content percentage (%) | sesame oil/ lactic acid | | |
| Ref. Ex. 26 | 4 | - | - | 2.04 (a) | - |
| Ex. 23 | 4 | 16 | 4 | 3.93 | (a)+(b)=2.52 |
| Ref. Ex. 27 | - | 16 | - | 0.48 (b) | |
| Ex. 24 | 4 | 24 | 6 | 4.74 | (a)+(c)=3.35 |
| Ref. Ex. 28 | - | 24 | - | 1.31 (c) | |
| Ex. 25 | 4 | 32 | 8 | 3.89 | (a)+(d)=3.13 |
| Ref. Ex. 29 | - | 32 | - | 1.09(d) | |
| Ex. 26 | 4 | 36 | 9 | 8.73 | (a)+(e)=3.83 |
| Ref. Ex. 30 | - | 36 | - | 1.79(e) | |
| Ex. 27 | 4 | 40 | 10 | 6.89 | (a)+(f)=3.44 |
| Ref. Ex. 31 | - | 40 | - | 1.40 (f) | |
| Ex. 28 | 4 | 44 | 11 | 9.73 | (a)+(g)=3.85 |
| Ref. Ex. 32 | - | 44 | - | 1.81 (g) | |
| Ex. 29 | 4 | 48 | 12 | 9.45 | (a)+(h)=5.45 |
| Ref. Ex. 33 | - | 48 | - | 3.41 (h) | |

As shown in Table 3, the permeation amount of compound A in the preparation of the present application containing lactic acid and sesame oil, which is one embodiment of a particular additive, was clearly higher than the total of the permeation amounts of compound A in the preparation of a Reference Example simply containing the same amount of lactic acid and the preparation of a Reference Example simply containing the same amount of sesame oil, at least when sesame oil is added up to 12 times the amount of lactic acid. This exhibits the superior feature of the present invention that a combination of lactic acid and the particular additive at least at the above-mentioned mixing ratio synergistically enhances the skin permeability of compound A.

### Example 30

An acrylic adhesive (DURO-TAK 387-2516 (registered trade mark), manufactured by National Starch & Chemical Ltd., solid content 41.5%) (3.90 g), ethyl acetate (1.2 mL), and α-monoisostearyl glyceryl ether and lactic acid each to a content percentage of 5% in an adhesive layer were mixed. To the mixture was added compound A such that its content percentage in the adhesive layer was 9%, and the mixture was sufficiently stirred. The obtained mixture was spread on the support such that the thickness of the adhesive layer after drying was about 60 µm, and the layer was dried at room temperature for 3 days. Then, a release liner was adhered to give a tape preparation.

### Example 31

A tape preparation was produced using diisopropyl adipate instead of α-monoisostearyl glyceryl ether in Example 30.

### Experimental Example 3

In the same manner as in Experimental Example 1 and using an in vitro diffusion cell, the skin permeability of compound A in the tape preparations obtained in Examples 30 and 31 through the abdominal skin of hairless rats was measured. The results are shown in Table 4.

**[Table 4]**

| | acrylic adhesive | additive | permeation amount of compound A in hairless rat skin permeation test (µg/cm²/24 hr) |
|---|---|---|---|
| Ex. 30 | DURO-TAK 387-2516 (registered trade mark) | lactic acid (5%) + α-monoisostearyl glyceryl ether (5%) | 18.0 |
| Ex. 31 | | lactic acid (5%) + diisopropyl adipate (5%) | 10.9 |

### Example 32

A styrene-isoprene-styrene block copolymer (Quintac 3421, manufactured by ZEON CORPORATION) (0.4 g), liquid paraffin (0.4 g), polybutene (HV-300, manufactured by Nippon Petroleum Refining Corporation) (0.3 g), alicyclic saturated hydrocarbon resin (ARKON P-100, manufactured by Arakawa Chemical Industries, Ltd.) (0.5 g), toluene (3.0 mL), and sesame oil and lactic acid each to a content percentage of 5% in an adhesive layer were mixed. To the mixture was added compound A such that its content percentage in the adhesive layer was 10%, and the mixture was sufficiently stirred. The obtained mixture was spread on the support such that the thickness of the adhesive layer after drying was about 60 µm, and the layer was dried at room temperature for 7 days. Then, a release liner was adhered to give a tape preparation.

### Industrial Applicability

According to the adhesive preparation of the present invention, the skin permeability can be remarkably enhanced by adding lactic acid, and an additive comprising a particular skin permeation enhancer. Since this enables downsizing of the adhesive preparation, a practically preferable adhesive preparation superior in the usability and economic efficiency can be provided.

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the appended claims.

This application is based on a patent application No. 2011-021197 filed in Japan, the contents of which are incorporated in full herein.

## Claims

1. An adhesive preparation comprising a support and an adhesive layer formed on one surface of the support, wherein the adhesive layer comprises (i) 2-(4-ethyl-1-piperazinyl)-4-(4-fluorophenyl)-5,6,7,8,9,10-hexahydrocycloocta[b]pyridine (hereinafter to be referred to as "compound A") or a physiologically acceptable acid addition salt thereof, (ii) an adhesive, (iii) lactic acid, and (iv) at least one kind of additive selected from the group consisting of polyethylene glycol mono-p-isooctylphenylether, diisopropyl adipate, α-monoisostearyl glyceryl ether, oleic acid, acetic acid, propylene glycol, sesame oil, lauromacrogol, crotamiton, sorbitan monostearate, cetyl lactate, lauryl alcohol, cetyl alcohol, diethyl sebacate, sorbitan sesquioleate, egg-yolk lecithin, propylene carbonate, liquid paraffin, polyoxyethylene hydrogenated castor oil, polyethylene glycol monostearate, octyl alcohol and benzyl alcohol.

2. The adhesive preparation according to claim 1, wherein the additive is at least one kind selected from the group consisting of polyethylene glycol mono-p-isooctylphenylether, diisopropyl adipate, α-monoisostearyl glyceryl ether, oleic acid, acetic acid, propylene glycol, sesame oil, and lauromacrogol.

3. The adhesive preparation according to claim 1 or 2, wherein the additive is at least one kind selected from the group consisting of diisopropyl adipate, α-monoisostearyl glyceryl ether, oleic acid, propylene glycol, sesame oil, and lauromacrogol.

4. The adhesive preparation according to any one of claims 1 - 3, wherein the additive comprises sesame oil.

5. The adhesive preparation according to any one of claims 1 - 4, wherein the content of lactic acid in the adhesive layer is 0.01 - 20 wt%.

6. The adhesive preparation according to any one of claims 1 - 5, wherein the additive is contained at a ratio of 0.5 - 20 parts by weight per 1 part by weight of the lactic acid.

7. The adhesive preparation according to claim 6, wherein the additive is contained at a ratio of 0.8 - 12 parts by weight per 1 part by weight of the lactic acid.

8. The adhesive preparation according to any one of claims 1 - 7, wherein the total content of lactic acid and the additive in the adhesive layer is 0.1 - 60 wt%.

9. The adhesive preparation according to any one of claims 1 - 8, wherein the adhesive is at least one kind selected from an acrylic adhesive, a rubber adhesive and a silicone adhesive.

10. The adhesive preparation according to claim 9, wherein the adhesive comprises an acrylic adhesive.

11. The adhesive preparation according to claim 9 or 10, wherein the acrylic adhesive is at least one kind selected from the group consisting of a (co)polymer mainly comprising (meth)acrylic acid alkyl ester, and a copolymer of (meth)acrylic acid alkyl ester and a functional monomer.

12. The adhesive preparation according to claim 9 or 10, wherein the adhesive comprises a rubber adhesive.

13. The adhesive preparation according to claim 9 or 12, wherein the rubber adhesive is at least one kind selected from the group consisting of a styrene-isoprene-styrene block copolymer and polyisobutylene.

14. The adhesive preparation according to any one of claims 1 - 13, wherein the adhesive layer comprises component (i) at a concentration of 0.5 - 40 wt% in terms of compound A.

15. The adhesive preparation according to any one of claims 1 - 14 whose target disease is schizophrenia.
